# EUROPEAN PATENT SPECIFICATION

(11) **EP 1 294 393 B1**
(45) Date of publication and mention of the grant of the patent: **05.10.2005**
(21) Application number: 01940840.0
(22) Date of filing: 25.06.2001
(51) Int. Cl.: A61K 38/46, A61K 48/00, G01N 33/50, A61P 9/10

(54) **GLYCOSYLPHOSPHATIDYLINOSITOL SPECIFIC PHOSPHOLIPASE D PROTEINS FOR THE TREATMENT AND DIAGNOSIS OF ATHERSCLEROSIS**
GLYKOSYLPHOSPHATIDYLINOSITOL SPEZIFISCHE PHOSPHOLIPASE D PROTEINE ZUR BEHANDLUNG UND DIAGNOSE VON ATHEROSKLEROSE
PROTEINES DE PHOPHOLIPASE D A SPECIFICITE GLYCOSYLPHOSPHATIDYLINOSITOL UTILES POUR LE TRAITEMENT OU LE DIAGNOSTIC DE L'ATHEROSCLEROSE

(30) Priority: 29.06.2000 GB 0015986
(43) Date of publication of application: 26.03.2003
(73) Proprietor: UNIVERSITY COLLEGE LONDON, London WC1E 6BT (GB)
(72) Inventor: SCHOFIELD, Julian, London, Greater London EC1V 7JA (GB); RADEMACHER, Thomas William, Oxford, Oxfordshire OX1 5EY (GB)
(74) Representative: Kiddle, Simon John
(86) International application number: PCT/GB2001/002803
(87) International publication number: WO 2002/002756

(56) References cited:
- WO-A-00/39285
- US-A- 5 418 147
- O'BRIEN KEVIN D ET AL: "Glycosylphosphatidylinositol-specific phospholipase D is expressed by macrophages in human atherosclerosis and colocalizes with oxidation epitopes." CIRCULATION, vol. 99, no. 22, 8 June 1999 (1999-06-08), pages 2876-2882, XP002185518 ISSN: 0009-7322 cited in the application
- MAGUIRE ET AL: "Glycosyl phosphatidyl inositol phospholipase D activity in human serum" ANNALS OF CLINICAL BIOCHEMISTRY, BRITISH MEDICAL ASSOCIATION, LONDON, GB, vol. 32, no. 1, January 1995 (1995-01), pages 74-78, XP000864653 ISSN: 0004-5632

## Description

### Field of the Invention

The present invention relates to the use of glycosylphosphatidylinositol specific phospholipase D (GPI-PLD) proteins, and in particular the use of GPI-PLD for the treatment or diagnosis of conditions characterised by an atherosclerotic process.

### Background of the Invention

Studies have shown that a number of cell surface proteins are attached to the cell membrane by covalent linkage to a glycosylphosphatidylinositol (GPI) anchor. It has been shown that the enzyme GPI-PLD cleaves the phosphodiester bond linking glycosylphosphatidylinositol to phosphatidic acid, thereby releasing anchored proteins.

GPI-PLD proteins are abundant in bovine and human serum. It is thought that they are primarily synthesised by the Langerhans cells of the pancreas and mast cells and released into the circulation and transported to target tissue in a complex with apolipoprotein-A1 (ApoA1) (Hoener et al 1993). The GPI-PLD enzyme is capable of hydrolysing glycosylphosphatidylinositol, by which a number of cell surface proteins are attached to cell membranes. GPI-PLD cleaves a phosphodiester bond, to liberate phosphatidic acid, in contrast to phospholipase C activity, which generates diacyl glycerol.

A further proposed function of GPI-PLD is to produce inositolphosphoglycans by cleavage of "free" GPIs in the plasma membrane in response to a growth factor or hormone binding to its receptor (Rademacher et al, 1994). This role for GPI-PLD has been demonstrated in basophils where IgE-dependent activation of these cells results in release of their granule contents, which include substances such as histamine, a mediator of the inflammatory response. In the presence of antigen, histamine is released; this release can be mimicked by addition of IPGs and is blocked by addition of anti-GPI-PLD antibodies (Lin et al, 1991).

US Patent No: 5,418,147 (Huang et al) describes the purification of GPI-PLD from bovine liver, and the subsequent cloning of three GPI-PLD enzymes from bovine liver, human liver and human pancreas cDNA libraries. This patent reports the full length cDNA and amino acid sequences of the GPI-PLDs from human and bovine liver, and the partial cDNA and amino acid sequences of the human pancreatic form of the enzyme. Subsequently, the full length sequence of the pancreatic form of GPI-PLD was reported in Tsang et al (1992), and this enzyme has been found in cDNA libraries from breast, eye, spleen and tonsil. The three forms of the enzymes are highly homologous with the predicted mature protein sequences of bovine liver GPI-PLD sharing 82% sequence identity with the human liver enzyme and 81% sequence identity with the human pancreatic enzyme. The amino acid sequences of human liver and pancreatic forms of GPI-PLD were deposited at GenBank under accession numbers L11701 and L11702 and consist of 841 and 840 amino acids respectively. The human liver and pancreatic forms of GPI-PLD share 94.6% sequence identity. The structure of GPI-PLDs is further discussed in Scallon et al, 1991.

However, despite the cloning of GPI-PLD enzymes and investigation as to their biochemical properties, many of the roles of the enzyme *in vivo* or any possible medical use remain unknown.

O'Brien et al (1999) relates to the expression of GPI-PLD by macrophages and suggests that increased expression of GPI-PLD participates in causing inflammation and pathogenesis in atherosclerosis.

### Summary of the Invention

Broadly, the present invention relates to the use of GPI-PLD for the prevention and treatment of conditions characterised by atherosclerosis. In some embodiments, this process may be caused by a failure to produce GPI-PLD, for example causing a lipidic disorder associated with type 1 diabetes, or deliver GPI-PLD, such as patients deficient in apolipoprotein-A1, or those patients with autoantibodies to either GPI-PLD or Apo-A1. In other embodiments, the atherosclerotic process may result from a loss in GPI-PLD activity, e.g. where there is a genetic modification of GPI-PLD either affecting its activity or delivery to a target tissue, thereby leading to the atherosclerotic process. Thus, in contrast to O'Brien et al (1999), the present invention proposes treating conditions characterised by atherosclerosis by administering GPI-PLD.

Examples of conditions characterised by atherosclerosis include a lipidic disorder associated with type 1 diabetes, conditions characterised by ApoA1 deficiency, cardiovascular disorders such as coronary artery disease (CAD), systemic lupus erythematosis (SLE) and Sjogren's syndrome.

Accordingly, in a first aspect the present invention provides the use of GPI-PLD for the preparation of a medicament for the treatment of conditions characterised by atherosclerosis.

In a further aspect, the present invention provides the use of a nucleic acid molecule encoding GPI-PLD for the preparation of a medicament for the treatment of conditions characterised by atherosclerosis.

In a further aspect, the present invention provides the use of a host cell capable of expressing and secreting GPI-PLD in the preparation of a medicament for the treatment of conditions characterised by atherosclerosis.

In one embodiment, the host cell is encapsulated, e.g. in a biocompatible polymer, so that the GPI-PLD produced by the host cell can be secreted into the patient, while preventing rejection of the host cell by the immune system of the patient. Methods for encapsulating cells in biocompatible polymers are described in WO93/16687 and WO96/31199.

In a further aspect, the present invention provides a method of diagnosing a patient who has or at risk of developing a condition characterised by atherosclerosis, the method comprising determining the amount of GPI-PLD and/or GPI-PLD activity in a sample obtained from the patient.

In one embodiment, the present invention provides a method of diagnosing a patient who has or at risk of developing a condition characterised by atherosclerosis, the method comprising the steps of:
(a) contacting a sample obtained from the patient with a solid support having immobilised thereon a binding agent having binding sites specific for GPI-PLD;
(b) determining the amount of GPI-PLD or the activity of GPI-PLD which binds to the binding agent.
   The method may involve the additional step of:
(c) correlating the value obtained in step (b) with measurements obtained from control subjects to determine whether the patient has or is at risk of developing the condition.

These and other aspects of the present invention are described in more detail below. By way of example, embodiments of the present invention will be described with reference to the accompanying figures.

### Brief Description of the Figures

Figure 1 shows an alignment of the deduced amino acid sequences of human liver GPI-PLD disclosed in PCT/GB99/04399 the bovine and human liver GPI-PLD sequences disclosed in US Patent No: 5,418,147 (Huang et al).
Figure 2 shows GPI-PLD plasma levels observed in samples from patients with autoimmmune disorders, as compared to control groups (normal patients and patients with rheumatoid arthritis).

### Detailed Description

### GPI-PLD Proteins

The term "GPI-PLD biological activity" is herein defined as the enzymatic activity of GPI-PLD in liberating phosphatidic acid from a glycosylphosphatidylinositol by cleavage of a phosphodiester bond, e.g. releasing a GPI-anchored protein. As noted in Heller et al (1994), this activity has been localised to the N-terminal 39 kD portion of full length GPI-PLD.

The medical uses of GPI-PLD described herein can employ the GPI-PLD variants disclosed by Huang et al, and in PCT/GB99/04399. The skilled person can use the techniques described herein and others well known in the art to produce large amounts of these proteins, or fragments, active portions, variants and alleles thereof, for use as pharmaceuticals.

GPI-PLD proteins which are amino acid sequence variants or alleles of these prior art sequences can also be used in the present invention. A polypeptide which is a variant or allele may have an amino acid sequence which differs from that given by one or more of addition, substitution, deletion and insertion of one or more amino acids. In some embodiments, the polypeptides have GPI-PLD enzymatic function as defined above. In other embodiments, the GPI-PLDs are enzymatically inactive, e.g. not having the activity of releasing IPGs, and can compete with the abnormal or dysregulated GPI-PLD to down regulate the abnormal GPI-PLD activity potentially leading to atherosclerosis.

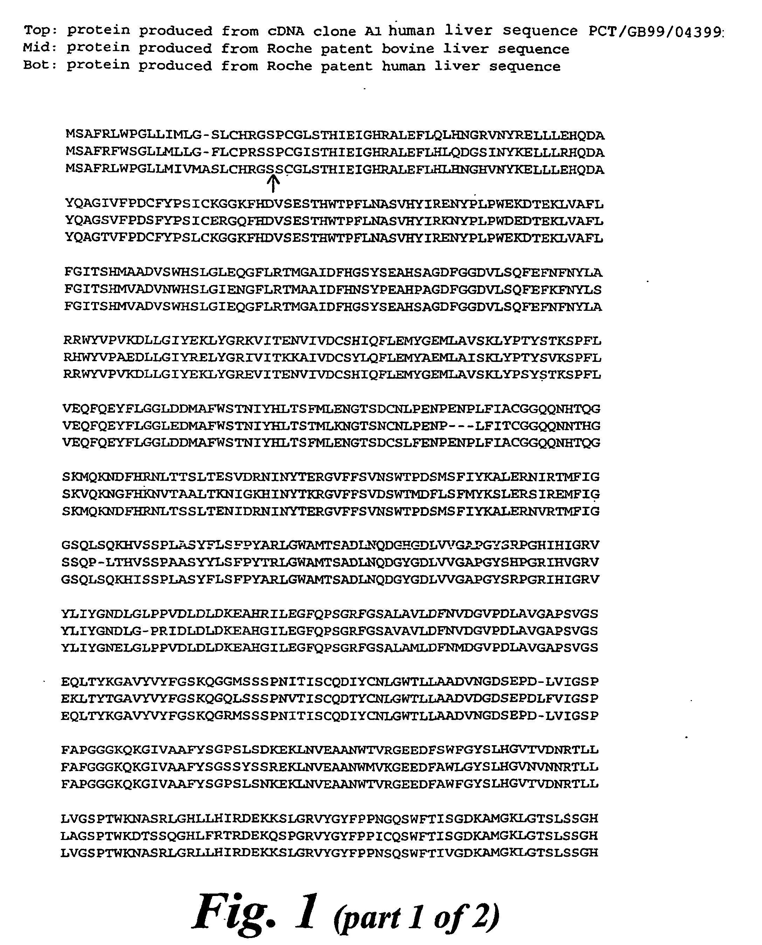

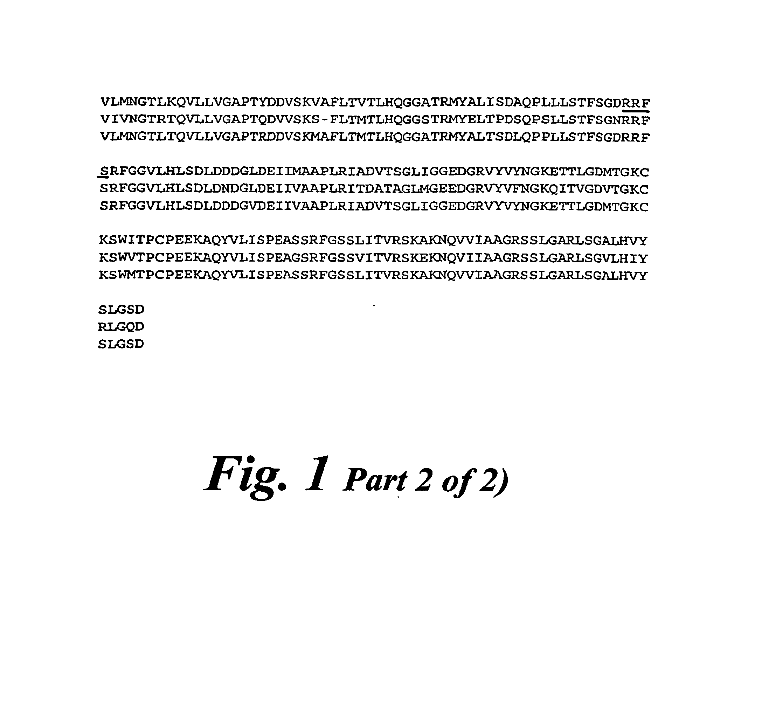

A GPI-PLD protein which is an amino acid sequence variant or allele of an amino acid sequence shown in Figure 1 may comprise an amino acid sequence which shares greater than about 70%, greater than about 80%, greater than about 90%, greater than about 95%, greater than about 97%, greater than about 98% or greater than about 99% sequence identity with an amino acid sequence shown in Figure 1. Sequence comparison and identity calculations may be carried out using the Cluster program (Thompson et al, 1994), using the following parameters (Pairwise Alignment Parameters: Weight Matrix: pam series; Gap Open Penalty: 10.00; Gap Extension Penalty: 0.10). Alternatively, the GCG program could be used which is available from Genetics Computer Group, Oxford Molecular Group, Madison, Wisconsin, USA, Version 9.1. Particular amino acid sequence variants may differ from those shown in Figure 1 by insertion, addition, substitution or deletion of 1 amino acid, 2, 3, 4, 5-10, 10-20 20-30, 30-50, 50-100, 100-150, or more than 150 amino acids.

In one embodiment, the variant GPI-PLD polypeptides of the present invention differ in amino acid sequence as compared to human GPI-PLD at the phosphorylation site from amino acids 689 to 692 of the mature sequence, i.e. within the amino acid motif RRFS. The term "variant GPI-PLD polypeptide" is intended, inter alia, to include polypeptides which are modified within this region by deletion, substitution and/or insertion of one or more amino acids. These sequence differences may be the result of varying the GPI-PLD amino acid sequence of a parent GPI-PLD polypeptide, either a wild type GPI-PLD polypeptide or a GPI-PLD polypeptide comprising one or more other modifications, e.g. by manipulation of the nucleic acid encoding the polypeptide, by altering the polypeptide itself or by the *de novo* synthesis of the variant protein. In preferred embodiments, the GPI-PLD retains, at least in part, one of its biological activities, e.g. by including a functional N-terminal domain.

A deletion may take the form of the deletion of one, two, three or all four amino acids within this region. In some embodiments, the deletion may be part of a larger deletion encompassing a greater part of the GPI-PLD molecule. In a preferred embodiment, the variant GPI-PLD polypeptides have an amino acid sequence which differs from the amino acid sequence of human wild type GPI-PLD by the deletion comprising residues 689 to 692 inclusive.

An insertion may take the form of 1, 2, 3, 4 or 5 or more additional amino acids inserted between amino acids within the RRFS motif to disrupt it.

A substitution may take the form of the substitution of one, two, three or all of the four amino acids within the region corresponding to amino acids 689 to 692 of wild type human GPI-PLD. The substitutions within this region may be part of a more extensive series of substitutions encompassing other parts of the GPI-PLD polypeptide. In particular, mutant forms of GPI-PLD which may have practical use differ from the wild type sequence. Some of these mutants are used in the experiments described below.

These sequence differences may be the result of varying the GPI-PLD amino acid sequence of a parent GPI-PLD polypeptide, either a wild type GPI-PLD polypeptide or a GPI-PLD polypeptide comprising one or more other modifications, e.g. by manipulation of the nucleic acid encoding the polypeptide, by altering the polypeptide itself or by the *de novo* synthesis of the variant protein. In preferred embodiments, the GPI-PLD retains, at least in part, one of its biological activities, e.g. by the presence of a functional N-terminal domain.

It is preferred that GPI-PLD variants for use in the present invention retain the enzymatic activity of GPI-PLD in liberating phosphatidic acid from a glycosylphosphatidylinositol by cleavage of a phosphodiester bond.

In other embodiments, the GPI-PLD may be a non-enzymatically active variant that may be used to treat atherosclerosis where the over activity of abnormal or dysregulated GPI-PLD is a causative factor of this condition. In this case, the non-enzymatically active variants can compete with or displace the abnormal or dysregulated GPI-PLD, e.g. from its Apo-A1 carrier, thereby having the effect of down regulating the GPI-PLD activity. The design and synthesis of such competitive antagonists can be readily carried out using the information about the GPI-PLD provided in this application.

The present invention also includes the use of active portions and fragments of the GPI-PLD proteins for the preparation of a medicament for the prevention or treatment of conditions characterised by atherosclerosis.

An "active portion" of GPI-PLD protein is a polypeptide which is less than said full length GPI-PLD protein, but which retains at least its essential enzymatic activity, e.g. the enzyme activity mentioned above known to be located in the N-terminal 39kD portion of the enzyme.
For instance, portions of GPI-PLD protein can act as sequestrators or competitive antagonists by interacting with other proteins. Typically, active portions comprise at least about 300 amino acids of GPI-PLD, more preferably at least 500 amino acids, and still more preervably at least 700 amino acids, with, for example, the amino acids being selected from one or more motifs from a sequence shown in Figure 1.

A "fragment" of the GPI-PLD protein means a stretch of amino acid residues of at least about 5 to 7 contiguous amino acids, often at least about 7 to 9 contiguous amino acids, typically at least about 9 to 13 contiguous amino acids, more preferably at least about 20 to 30 or more contiguous amino acids, more preferably greater than 40 amino acids, more preferably greater than 100 amino acids.

A polypeptide for use in the preparation of a medicament for the prevention or treatment of atherosclerotic conditions according to the present invention may be isolated and/or purified (e.g. using an antibody) for instance after production by expression from encoding nucleic acid (for which see below). Polypeptides according to the present invention may also be generated wholly or partly by chemical synthesis.

The GPI-PLD polypeptides can also be linked to a coupling partner, e.g. an effector molecule, a label, a drug, a toxin and/or a carrier or transport molecule. Techniques for coupling the peptides of the invention to both peptidyl and non-peptidyl coupling partners are well known in the art. In one embodiment, the carrier molecule is a 16 aa peptide sequence derived from the homeodomain of *Antennapedia* (e.g. as sold under the name "Penetratin"), which can be coupled to a peptide via a terminal Cys residue. The "Penetratin" molecule and its properties are described in WO91/18981.

### GPI-PLD Nucleic Acid

"GPI-PLD nucleic acid" includes a nucleic acid molecule which has a nucleotide sequence encoding a polypeptide with GPI-PLD activity. Suitable full-length sequences of human and bovine GPI-PLD enzymes have been provided, for example, by Huang et al, Tsang et al (1992) and in PCT/GB99/04399. These forms of GPI-PLD have been mapped to human chromosome 6 and are contained in the 4 centimorgan region of D6S1660-D6S1558 at positions 95.95 and 99.71 (NCBI GeneMap'98). The gene starts in the cytogenic region corresponding to 6p22.3 and extends into 6p21.3. This region also contains the IDDM1 and HLA loci (although the HLA genes map to the adjacent D6S1558-D6S1616 interval). The mouse GPI-PLD gene has also been mapped to chromosome 13, near the *fim 1* locus, which is found in humans on chromosome 6.

The GPI-PLD coding sequence may be one of the published sequences referred to above, or a complementary nucleic acid sequence, or it may be a mutant, variant, derivative or allele of one of these sequences. The sequence may differ from that shown by a change which is one or more of addition, insertion, deletion and substitution of one or more nucleotides of the sequence shown. Changes to a nucleotide sequence may result in an amino acid change at the protein level, or not, as determined by the genetic code.

The encoded polypeptide may comprise an amino acid sequence which differs by one or more amino acid residues from one of the published GPI-PLD amino acid sequences. Use of nucleic acid encoding a polypeptide which is an amino acid sequence mutant, variant or allele of a known GPI-PLD is further envisaged by the present invention. Such polypeptides are discussed below. Nucleic acid encoding such a polypeptide may show greater than about 70% identity, greater than about 80% identity, greater than about 90% identity, greater than about 95% identity, greater than about 98% identity, or greater than about 99% identity with a published coding sequence for a GPI-PLD protein as described above.

The present invention also embraces use of fragments of GPI-PLD nucleic acid sequences, the fragments preferably being at least 12, 15, 30, 45, 60, 120 or 240 nucleotides in length.

Generally, nucleic acid for use according to the present invention is provided as an isolate, in isolated and/or purified form, or free or substantially free of material with which it is naturally associated, such as free or substantially free of nucleic acid flanking the gene in the human genome, except possibly one or more regulatory sequence(s) for expression. Nucleic acid may be wholly or partially synthetic and may include genomic DNA, cDNA or RNA. Where nucleic acid according to the invention includes RNA, reference to the sequence shown should be construed as reference to the RNA equivalent, with U substituted for T.

Nucleic acid sequences encoding all or part of the GPI-PLD gene and/or its regulatory elements can be readily prepared by the skilled person using the information and references contained herein and techniques known in the art. For example, see Sambrook et al, 1989, and Ausubel et al, 1992. These techniques include (i) the use of the polymerase chain reaction (PCR) to amplify samples of such nucleic acid, e.g. from genomic sources, (ii) chemical synthesis, or (iii) amplification in *E. coli.* Modifications to the GPI-PLD sequences can be made, e.g. using site directed mutagenesis, to provide expression of modified GPI-PLD protein or to take account of codon preference in the host cells used to express the nucleic acid.

In order to obtain expression of the GPI-PLD nucleic acid sequences, the sequences can be incorporated in a vector having control sequences operably linked to the GPI-PLD nucleic acid to control its expression. The use of expression systems has reached an advanced degree of sophistication. The vectors may include other sequences such as promoters or enhancers to drive the expression of the inserted nucleic acid, nucleic acid sequences so that the GPI-PLD protein is produced as a fusion and/or nucleic acid encoding secretion signals so that the polypeptide produced in the host cell is secreted from the cell. GPI-PLD protein can then be obtained by transforming the vectors into host cells in which the vector is functional, culturing the host cells so that the GPI-PLD protein is produced and recovering the GPI-PLD protein from the host cells or the surrounding medium. Prokaryotic and eukaryotic cells are used for this purpose in the art, including strains of *E. coli*, yeast, and eukaryotic cells such as COS or CHO cells.
The choice of host cell can be used to control the properties of the GPI-PLD protein expressed in those cells, e.g. controlling where the polypeptide is deposited in the host cells or affecting properties such as its glycosylation and phosphorylation.

PCR techniques for the amplification of nucleic acid are described in US Patent No:4,683,195. In general, such techniques require that sequence information from the ends of the target sequence is known to allow suitable forward and reverse oligonucleotide primers to be designed to be identical or similar to the polynucleotide sequence that is the target for the amplification. PCR comprises steps of denaturation of template nucleic acid (if double-stranded), annealing of primer to target, and polymerisation. The nucleic acid probed or used as template in the amplification reaction may be genomic DNA, cDNA or RNA. PCR can be used to amplify specific sequences from genomic DNA, specific RNA sequences and cDNA transcribed from mRNA, bacteriophage or plasmid sequences. The published GPI-PLD protein nucleic acid sequences readily allow the skilled person to design PCR primers. References for the general use of PCR techniques include Mullis et al, Cold Spring Harbor Symp. Quant. Biol., 51:263, 1987; Ehrlich (ed), PCR Technology, Stockton Press, NY, 1989; Ehrlich et al, Science, 252:1643-1650, 1991; "PCR protocols; A Guide to Methods and Applications", Eds. Innis et al, Academic Press, New York, 1990.

Nucleic acid for use according to the present invention is obtainable using one or more oligonucleotide probes or primers designed to hybridize with one or more fragments of the nucleic acid sequence shown in the figures, particularly fragments of relatively rare sequence, based on codon usage or statistical analysis. A primer designed to hybridize with a fragment of the nucleic acid sequence shown in the above figures may be used in conjunction with one or more oligonucleotides designed to hybridize to a sequence in a cloning vector within which target nucleic acid has been cloned, or in so-called "RACE" (rapid amplification of cDNA ends) in which cDNA's in a library are ligated to an oligonucleotide linker and PCR is performed using a primer which hybridizes with a GPI-PLD nucleic acid sequence shown in figures and a primer which hybridizes to the oligonucleotide linker.

Such oligonucleotide probes or primers, as well as the full-length sequence (and mutants, alleles, variants and derivatives) are also useful in screening a test sample containing nucleic acid for the presence of alleles, mutants and variants of GPI-PLD protein, the probes hybridizing with a target sequence from a sample obtained from the individual being tested. The conditions of the hybridization can be controlled to minimise non-specific binding, and preferably stringent to moderately stringent hybridization conditions are preferred. The skilled person is readily able to design such probes, label them and devise suitable conditions for the hybridization reactions, assisted by textbooks such as Sambrook et al (1989) and Ausubel et al (1992).

Examples of "stringent conditions" are those which: (1) employ low ionic strength and high temperature for washing, for example 0.015 M sodium chloride/0.0015 M sodium citrate/0.1% sodium dodecyl sulphate at 50°C; (2) employ during hybridisation a denaturing agent, such as formamide, for example, 50% (v/v) formamide with 0.1% BSA/0.1% Ficoll/0.1% polyvinylpyrrolidone/50mM sodium phosphate buffer at pH 6.5 with 750mM sodium chloride, 75mM sodium citrate at 42°C; or (3) employ 50% formamide, 5 x SSC (0.75 M NaCl, 0.075 M sodium citrate), 50mM sodium phosphate (pH 6.8), 0.1% sodium pyrophosphate, 5 x Denhardt's solution, sonicated salmon sperm DNA (50mg/ml), 0.1% SDS, and 10% dextran sulphate at 42°C, with washes at 42°C in 0.2 x SSC and 50% formamide at 55°C, followed by high stringency wash consisting of 0.1 x SSC containing EDTA at 55°C. These hybridisation conditions may be used in the context of defining nucleic acid sequences which hybridize with GPI-PLD nucleic acid sequences.

### Uses of GPI-PLD Nucleic Acid

The GPI-PLD nucleic acid sequences can be used in the preparation of cell lines capable of expressing GPI-PLD and included in expression vectors or otherwise formulated, e.g. for use in gene therapy techniques.

Thus, the present invention provides a cell line for transplantation into a patient, the cell line being transformed with nucleic acid encoding GPI-PLD, and being capable of expressing and secreting GPI-PLD. In one embodiment, the cell lines are encapsulated, e.g. in a biocompatible polymer, so that the GPI-PLD produced by the cell line can be secreted into the patient, while preventing rejection by the immune system of the host. Methods for encapsulating cells in biocompatible polymers are described in WO93/16687 and WO96/31199.

As a further alternative, the nucleic acid encoded the GPI-PLD protein could be used in a method of gene therapy, to treat a patient who is unable to synthesize the active polypeptide or unable to synthesize it at the normal level, thereby providing the effect provided by wild-type GPI-PLD protein and suppressing the occurrence of diabetes in the target cells.

Vectors such as viral vectors have been used in the prior art to introduce genes into a wide variety of different target cells. Typically, the vectors are exposed to the target cells so that transfection can take place in a sufficient proportion of the cells to provide a useful therapeutic or prophylactic effect from the expression of the desired polypeptide. The transfected nucleic acid may be permanently incorporated into the genome of each of the targeted tumour cells, providing long lasting effect, or alternatively the treatment may have to be repeated periodically.

A variety of vectors, both viral vectors and plasmid vectors, are known in the art, see US Patent No: 5,252,479 and WO93/07282. In particular, a number of viruses have been used as gene transfer vectors, including papovaviruses, such as SV40, vaccinia virus, herpesviruses, including HSV and EBV, and retroviruses. Many gene therapy protocols in the prior art have used disabled murine retroviruses.

As an alternative to the use of viral vectors other known methods of introducing nucleic acid into cells includes electroporation, calcium phosphate co-precipitation, mechanical techniques such as microinjection, transfer mediated by liposomes and direct DNA uptake and receptor-mediated DNA transfer.

As mentioned above, the aim of gene therapy using nucleic acid encoding the GPI-PLD protein, or an active portion thereof, is to increase the amount of the expression product of the nucleic acid in cells in which the level of the wild-type GPI-PLD protein is absent or present only at reduced levels. Target cells for gene therapy include insulin secreting β-cells or any neuron derived cells. Cell engineering can be used to provide the overexpression or repression of GPI-PLD protein in transfected cell lines which can then be subsequently transplanted to humans. Gene therapy can be employed using a promoter to drive GPI-PLD protein expression in a tissue specific manner (i.e. an insulin promoter linked to GPI-PLD cDNA will overexpress GPI-PLD protein in b-cells and transiently in the brain). If defective function of GPI-PLD protein is involved in neurological disease, GPI-PLD protein can be overexpressed in transformed cell lines for transplantation.

Gene transfer techniques which selectively target the GPI-PLD nucleic acid to target tissues are preferred. Examples of this included receptor-mediated gene transfer, in which the nucleic acid is linked to a protein ligand via polylysine, with the ligand being specific for a receptor present on the surface of the target cells.

### Pharmaceutical Compositions

As mentioned above, GPI-PLD proteins and nucleic acid can be formulated as pharmaceutical compositions using techniques well known in the art.

Pharmaceutical compositions for oral administration may be in tablet, capsule, powder or liquid form. A tablet may include a solid carrier such as gelatin or an adjuvant or an inert diluent. Liquid pharmaceutical compositions generally include a liquid carrier such as water, petroleum, animal or vegetable oils, mineral oil or synthetic oil. Physiological saline solution, or glycols such as ethylene glycol, propylene glycol or polyethylene glycol may be included. Such compositions and preparations generally contain at least 0.1 wt% of the compound.

Parenteral administration includes administration by the following routes: intravenous, cutaneous or subcutaneous, nasal, intramuscular, intraocular, transepithelial, intraperitoneal and topical (including dermal, ocular, rectal, nasal, inhalation and aerosol), and rectal systemic routes. For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, solutions of the compounds or a derivative thereof, e.g. in physiological saline, a dispersion prepared with glycerol, liquid polyethylene glycol or oils.

In addition to one or more of the compounds, optionally in combination with other active ingredient, the compositions can comprise one or more of a pharmaceutically acceptable excipient, carrier, buffer, stabiliser, isotonicizing agent, preservative or antioxidant or other materials well known to those skilled in the art. Such materials should be non-toxic and should not interfere with the efficacy of the active ingredient. The precise nature of the carrier or other material may depend on the route of administration, e.g. orally or parenterally.

Liquid pharmaceutical compositions are typically formulated to have a pH between about 3.0 and 9.0, more preferably between about 4.5 and 8.5 and still more preferably between about 5.0 and 8.0. The pH of a composition can be maintained by the use of a buffer such as acetate, citrate, phosphate, succinate, Tris or histidine, typically employed in the range from about 1 mM to 50 mM. The pH of compositions can otherwise be adjusted by using physiologically acceptable acids or bases.

Preservatives are generally included in pharmaceutical compositions to retard microbial growth, extending the shelf life of the compositions and allowing multiple use packaging. Examples of preservatives include phenol, meta-cresol, benzyl alcohol, para-hydroxybenzoic acid and its esters, methyl paraben, propyl paraben, benzalconium chloride and benzethonium chloride. Preservatives are typically employed in the range of about 0.1 to 1.0 % (w/v).

Preferably, the pharmaceutically compositions are given to an individual in a "prophylactically effective amount" or a "therapeutically effective amount" (as the case may be, although prophylaxis may be considered therapy), this being sufficient to show benefit to the individual. Typically, this will be to cause a therapeutically useful activity providing benefit to the individual. The actual amount of the compounds administered, and rate and time-course of administration, will depend on the nature and severity of the condition being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 16th edition, Osol, A. (ed), 1980. By way of example, and the compositions are preferably administered to patients in dosages of between about 0.01 and 100mg of active compound per kg of body weight, and more preferably between about 0.5 and 10mg/kg of body weight.

The composition may further comprise one or more other pharmaceutically active agents, either further compounds of the invention, inositol phosphoglycans, growth factors such as insulin, NGF or other growth factors listed below, or other drugs, e.g. those in use for the treatment of the conditions set out below.

### Medical Uses

The GPI-PLD polypeptides and nucleic acid of the invention can be used in the preparation of medicaments to treat (either prophylactically or therapeutically) conditions characterised by atherosclerosis, or autoimmune conditions, especially those characterised by the presence of autoantibodies to GPI-PLD or the ApoAl/GPI-PLD complex. Examples of conditions include cardiovascular disorders and their complications such as coronary artery disease (CAD), the treatment of systemic lupus erythematosis (SLE), especially atherosclerotic lesions, Sjogren's syndrome and lipidic disorders associated with Type 1 or Type 2 diabetes.

Dyslipidemia is recognised as a classic risk factor in coronary artery disease. Premature atherosclerosis is an important cause of morbity in patients with systemic lupus erythematosis (SLE), an autoimmune disease of unknown cause with a wide spectrum of systemic pathology. It has been reported that patients with SLE contain autoantibodies directed against apolipoprotein A1 (Merrill et al, Arthitis and Rheum., 38:1655-1659, 1995).

Without wishing to be bound by any particular theory, the present invention postulates that in patients having conditions characterised by atherosclerosis have a defect in the GPI-PLD delivery by ApoA1. This is consistent with patients who have a congenital absence of Apo A1 and exhibit diabetic complications.

In some aspects, the GPI-PLD may be administered in combination with apolipoprotein A1 to which it is bound in human serum and blood.

In a further aspect, the present invention provides an article of manufacture containing materials useful in the diagnosis and/or treatment of conditions characterised by atherosclerosis. The article of manufacture comprises a container and a label. Suitable containers include bottles, vials, syringes and test tubes, for example formed from materials such as glass or plastic. The container holds a composition which is effective for diagnosing or treating atherosclerosis and may have a sterile access port, e.g. a stopper which can be pierced by a needle to allow access to the composition. Where the article is used for the prevention or treatment of atherosclerosis, the composition may comprises GPI-PLD, or a variant, active portion or fragment thereof. The article may optionally comprise one or more further active ingredients, in admixture with the GPI-PLD or as part of a kit in one or more further containers, for the treatment of atherosclerosis or to improve the pharmacological properties of the GPI-PLD. Where the article is used for the diagnosis of atherosclerosis, the container may be used to hold one or more reagents useful in carrying out the assays described herein. The label on or associated with the container indicates the use of the composition for the diagnosis or treatment of atherosclerosis and may provide detailed instructions to direct the user. The article for manufacture may further comprise a second container comprising a pharmaceutically acceptable buffer, Ringer's solution and/or dextrose solution.

### Diagnostic Assays

Methods for determining GPI-PLD activity or the amount or concentration of this enzyme in biological samples from individuals are well known in the art and can be employed in the context of the present invention to determine whether an individual has or is at risk of developing atherosclerosis or one of the other conditions discussed herein. The purpose of such analysis may be used for diagnosis or prognosis to assist a physician in determining the severity or likely course of the atherosclerosis and/or to optimise treatment of it.

Preferred diagnostic methods rely on the determination of GPI-PLD activity as abnormal or dysregulated GPI-PLD activity present in the placenta is believed to be one of the causes of atherosclerosis. In one embodiment, this method comprises determining GPI-PLD activity in a biological sample obtained from the patient, e.g. by measuring the action of GPI-PLD on a labelled substrate. Alternatively or additionally, the concentration or amount of GPI-PLD in a sample may be determined. Typically, the methods employ biological samples such as blood, serum, tissue samples or urine. Depending on the sample, it may be advantageous to carry out a pretreatment step, e.g. to remove cellular debris or unwanted contaminants from the sample.

In some embodiments, the assay methods for determining GPI-PLD activity employ a binding agent having binding sites capable of specifically binding to GPI-PLD in individuals are well known in the art and can be employed in the context of the present invention to determine whether an individual has or is at risk of developing atherosclerosis or one of the other conditions discussed herein. The purpose of such analysis may be used for diagnosis or prognosis to assist a physician in determining the severity or likely course of the atherosclerosis and/or to optimise treatment of it.

Preferred diagnostic methods rely on the determination of GPI-PLD activity as abnormal or dysregulated GPI-PLD activity present in the placenta is believed to be one of the causes of atherosclerosis. In one embodiment, this method comprises determining GPI-PLD activity in a biological sample obtained from the patient, e.g. by measuring the action of GPI-PLD on a labelled substrate. Alternatively or additionally, the concentration or amount of GPI-PLD in a sample may be determined. Typically, the methods employ biological samples such as blood, serum, tissue samples or urine. Depending on the sample, it may be advantageous to carry out a pretreatment step, e.g. to remove cellular debris or unwanted contaminants from the sample.

In some embodiments, the assay methods for determining GPI-PLD activity employ a binding agent having binding sites capable of specifically binding to GPI-PLD in preference to other molecules, and especially other molecules likely to be present in the sample. Examples of binding agents include antibodies, receptors and other molecules capable of specifically binding GPI-PLD. Conveniently, the binding agent is immobilised on solid support, e.g. at a defined location, to make it easy to manipulate during the assay. This can be achieved using techniques well known in the art such as physisorption or chemisorption, e.g. employing biotin/avidin or biotin/streptavidin to chemically link the binding agent to the solid support.

The sample is generally contacted with a binding agent under appropriate conditions so that GPI-PLD present in the sample can bind to the binding agent. GPI-PLD activity can be determined by employing a suitable substrate, such as glycosylphosphatidylinositol which has a phosphodiester bond cleaved by GPI-PLD to release phosphatidic acid and a GPI-anchor, and monitoring the enzymatic reaction, e.g. by following the amount of substrate or reaction product(s), in the assay system. This process can be facilitated by labelling the substrate, and detecting the reduction in the label on the substrate or the increase of the label in a reaction product.

In the case of determinations of the amount of GPI-PLD in the sample (rather than its activity), the fractional occupancy of the binding sites of the binding agent can then be determined using a developing agent or agents. The developing agent can be used in a competitive method in which the developing agent competes with the analyte for occupied binding sites of the binding agent (e.g. using a labelled analogue of the analyte), or non-competitive method, in which the labelled developing agent binds analyte bound by the binding agent or to occupied binding sites (e.g. using an antibody with appropriate binding specificity). Both methods provide an indication of the number of the binding sites occupied by the analyte, and hence the concentration of the analyte in the sample, e.g. by comparison with standards obtained using samples containing known concentrations of the analyte.

Typically, the substrates or developing agents are tagged with a label or reporter molecule which are directly or indirectly generate detectable, and preferably measurable, signals. The linkage of reporter molecules may be directly or indirectly, covalently, e.g. via a peptide bond or non-covalently. Linkage via a peptide bond may be as a result of recombinant expression of a gene fusion encoding antibody and reporter molecule. Any method known in the art for separately conjugating the antibody to the detectable moiety may be employed, including those methods described by Hunter et al, Nature 144:945, 1962; David et al, Biochemistry 13:1014, 1974; Pain et al, J. Immunol. Meth. 40:219, 1981; and Nygren, J Histochem. and Cytochem. 30:407, 1982.

One favoured mode is to covalently link of each species of substrate or developing agent with an individual fluorochrome, phosphor or laser dye with spectrally isolated absorption or emission characteristics.
Suitable fluorochromes include fluorescein, rhodamine, luciferin, phycoerythrin and Texas Red. Suitable chromogenic dyes include diaminobenzidine. Other detectable labels include radioactive isotopic labels, such as ³H, ¹⁴C, ³²P, ³⁵S, ¹²⁶I, or ^{99m}Tc, and enzyme labels such as alkaline phosphatase, β-galactosidase or horseradish peroxidase, which catalyze reactions leading to detectable reaction products and can provide amplification of signal.

Other reporters include macromolecular colloidal particles or particulate material such as latex beads that are coloured, magnetic or paramagnetic, and biologically or chemically active agents that can directly or indirectly cause detectable signals to be visually observed, electronically detected or otherwise recorded. These molecules may be enzymes which catalyze reactions that develop or change colours or cause changes in electrical properties, for example. They may be molecularly excitable, such that electronic transitions between energy states result in characteristic spectral absorptions or emissions. They may include chemical entities used in conjunction with biosensors.

As mentioned above, the substrates or developing agents are labelled (e.g. with radioactive, fluorescent or enzyme labels) so that they can be detected using techniques well known in the art. Thus, radioactive labels can be detected using a scintillation counter or other radiation counting device, fluorescent labels using a laser and confocal microscope, and enzyme labels by the action of an enzyme label on a substrate, typically to produce a colour change.

### Examples

It has been established in SLE patients that autoantibodies are present against Apo-A1 and SLE is considered to be a prime model for premature coronary heart diease. The linkage to coronary heart disease has not been established in the prior art, but antibodies against the ApoA1-complex could interfere with the delivery of GPI-PLD to the tissues, having a similar clinical effect to the Apo-A1 deficient patients. These linkages suggest that deficiencies of GPI-PLD, in addition to mediating insulin resistance, also mediate deficiencies of ther IPG mediated growth factors whose diminished activity gives rise to coronary heart disease. There is therefore a linkage between diabetic complications, SLE and congenitaal disorders of Apo-A1 as all involve a reuction in GPI-PLD levels in target tissues.

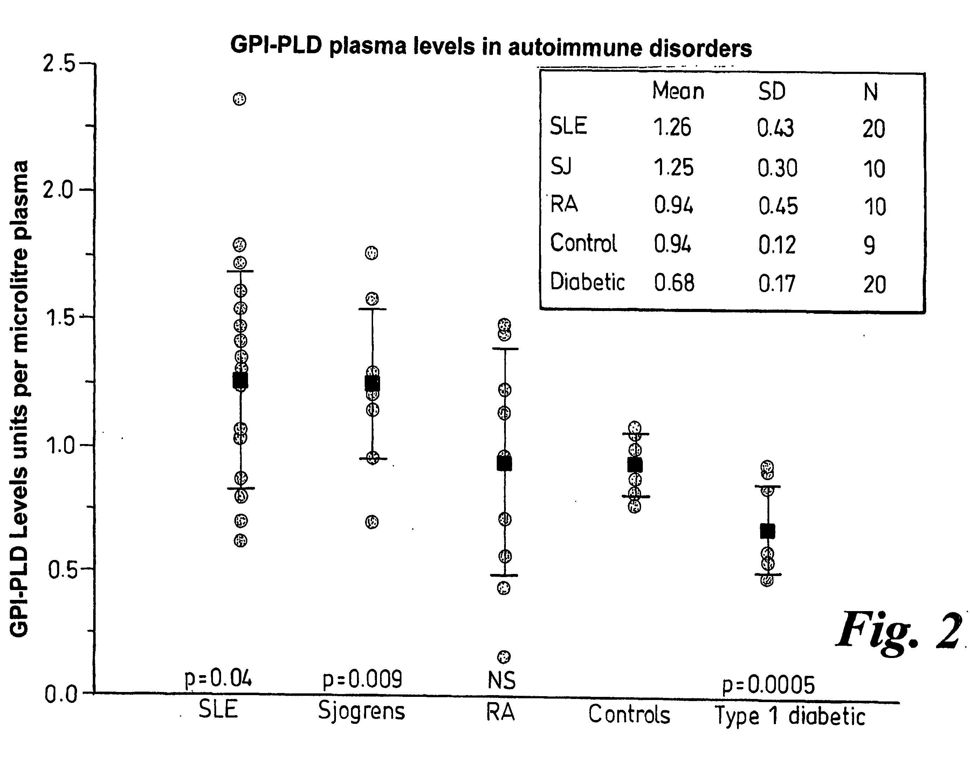

In order to test this hypothesis, a comparison was made of GPI-PLD plasma levels in a number of autoimmune disorders. The GPI-PLD levels in patients with SLE and Sjogren's syndrome was measured and compared to control groups, namely normal patients and patients with rheumatoid arthritis. As shown in Figure 2, the GPI-PLD levels in patients with both SLE and Sjogren's syndrome were statistically elevated, consistent with the disclosure herein that, in these conditions, autoantibodies inhibit or prevent delivery of GPI-PLD to the tissues and lead to a build-up of GPI-PLD in the blood stream. Thus, this creates a paradoxical deficiency in that such patients have high plasma levels of GPI-PLD and corresponding deficiency in tissue.

### References:

Huang et al, US Patent No: 5,418,147.
Tsang et al, FASEB J. (supp), 6:A1922, 1992.
Scallon et al, Science, 252:446-448, 1991.
Hoener et al, Eur. J. Biochem., 206:747-757, 1992.
Heller et al, Eur. J. Biochem., 224:823-833, 1994.
Rademacher et al, Brazilian J. Med. Biol. Res., 27:327-341, 1994.
Lin et al, J. Cell Biol., 115:220a, 1991.
Thompson et al, Nucleic Acid Research, 22:4673-4680, 1994, with algorithm from Higgins et al, CABIOS, 8(2):189-191, 1992.
Caro et al, Biochem. Molec. Med., 61:214-228, 1997.
O'Brien et al, Circulation, 99: 2876-2882, 1999.
Merrill et al, Arthritis & Rheumatism, 38:1655-1659, 1995.
Borba et al, Arthritis & Rheumatism, 43: 1033-1040, 2000.

## Claims

1. Use of GPI-PLD for the preparation of a medicament for the treatment of conditions **characterised by** atherosclerosis.

2. Use of a nucleic acid molecule encoding GPI-PLD for the preparation of a medicament for the treatment of conditions **characterised by** atherosclerosis.

3. The use of claim 1 or claim 2, wherein the atherosclerosis is caused by a failure to produce GPI-PLD, a failure to deliver GPI-PLD, or by the presence of autoantibodies to either GPI-PLD or Apo-A1.

4. The use of claim 1 or claim 2, wherein the atherosclerosis is the result of an autoimmune condition.

5. The use of claim 1 or claim 2, wherein the atherosclerosis results from a loss in GPI-PLD activity.

6. Use of a host cell capable of expressing and secreting GPI-PLD in the preparation of a medicament for the treatment of conditions **characterised by** atherosclerosis.

7. The use of any one of the preceding claims, wherein the condition is a lipidic disorder associated with type 1 diabetes, a condition **characterised by** ApoA1 deficiency, a cardiovascular disorders such as coronary artery disease (CAD), systemic lupus erythematosis (SLE) or Sjogren's syndrome.

8. A method of diagnosing a patient who has or at risk of developing a condition **characterised by** atherosclerosis, the method comprising determining the amount of GPI-PLD and/or GPI-PLD activity in a sample obtained from the patient.

9. The method of claim 8, wherein the method comprises the steps of:
(a) contacting a sample obtained from the patient with a solid support having immobilised thereon a binding agent having binding sites specific for GPI-PLD;
(b) determining the amount of GPI-PLD or the activity of GPI-PLD which binds to the binding agent.

10. The method of claim 9, wherein the method further comprises the step of:
(c) correlating the value obtained in step (b) with measurements obtained from control subjects to determine whether the patient has or is at risk of developing the condition.

11. The method of any one of claims 8 to 10, wherein the condition is a lipidic disorder associated with type 1 diabetes, a condition **characterised by** ApoA1 deficiency, a cardiovascular disorders such as coronary artery disease (CAD), systemic lupus erythematosis (SLE) or Sjogren's syndrome.

## Patentansprüche

1. Verwendung von GPI-PLD zur Herstellung eines Medikaments zur Behandlung von Leiden, die durch Atherosklerose **gekennzeichnet** sind.

2. Verwendung eines Nucleinsäuremoleküls, das für GPI-PLD kodiert, zur Herstellung eines Medikaments zur Behandlung von Leiden, die durch Atherosklerose **gekennzeichnet** sind.

3. Verwendung nach Anspruch 1 oder Anspruch 2, worin die Atherosklerose durch einen Ausfall der Produktion von GPI-PLD, durch einen Ausfall der Zufuhr von GPI-PLD oder durch die Gegenwart von Autoantikörpern gegen entweder GPI-PLD oder Apo-A1 verursacht wird.

4. Verwendung nach Anspruch 1 oder Anspruch 2, worin die Atherosklerose das Resultat eines Autoimmunleidens ist.

5. Verwendung nach Anspruch 1 oder Anspruch 2, worin die Atherosklerose aus einem Verlust an GPI-PLD-Aktivität resultiert.

6. Verwendung einer Wirtszelle, die in der Lage ist, GPI-PLD zu exprimieren und zu sekretieren, zur Herstellung eines Medikaments zur Behandlung von Leiden, die durch Atherosklerose **gekennzeichnet** sind.

7. Verwendung nach einem der vorangehenden Ansprüche, worin das Leiden eine Lipidstörung in Verbindung mit Diabetes vom Typ 1, ein Leiden, das durch ApoA1-Defizienz **gekennzeichnet** ist, eine kardiovaskuläre Störung wie beispielsweise koronare Herzkrankheit (CAD), systemischer Lupus erythematodes (SLE) oder Sjörgen-Syndrom ist.

8. Verfahren zur Diagnose eines Patienten, der an einem Leiden, das durch Atherosklerose **gekennzeichnet** ist, erkrankt oder gefährdet ist, ein solches zu entwickeln, worin das Verfahren das Bestimmen der Menge an GPI-PLD und/oder der GPI-PLD-Aktivität in einer dem Patienten entnommenen Probe umfasst.

9. Verfahren nach Anspruch 8, worin das Verfahren die folgenden Schritte umfasst:
(a) Kontaktieren einer dem Patienten entnommenen Probe mit einem festen Träger, auf dem ein Bindungsmittel mit Bindungsstellen, die für GPI-PLD spezifisch sind, immobilisiert ist;
(b) Bestimmen der Menge von GPI-PLD oder der Aktivität von GPI-PLD, das sich an das Bindungsmittel bindet.

10. Verfahren nach Anspruch 9, worin das Verfahren weiters den folgenden Schritt umfasst:
(c) Korrelieren des in Schritt (b) erhaltenen Werts mit Messungen, die von Kontrollsubjekten erhalten wurden, um zu bestimmen, ob der Patient an dem Leiden erkrankt oder gefährdet ist, dieses zu entwickeln.

11. Verfahren nach einem der Ansprüche 8 bis 10, worin das Leiden eine Lipidstörung in Verbindung mit Diabetes vom Typ 1, ein Leiden, das durch ApoA1-Defizienz **gekennzeichnet** ist, eine kardiovaskuläre Störung wie beispielsweise koronare Herzkrankheit (CAD), systemischer Lupus erythematodes (SLE) oder Sjörgen-Syndrom ist.

## Revendications

1. Utilisation de GPI -PLD pour la préparation d'un médicament pour le traitement de conditions **caractérisées par** l'athérosclérose.

2. Utilisation d'une molécule d'acide nucléique codant pour GPI -PLD pour la préparation d'un médicament pour le traitement de conditions **caractérisées par** l'athérosclérose.

3. Utilisation de la revendication 1 ou de la revendication 2, où l'athérosclérose est provoquée par un défaut de production de GPI-PLD, un défaut de délivrance de GPI-PLD, ou par la présence d'auto-anticorps contre GPI-PLD ou Apo-A1.

4. Utilisation de la revendication 1 ou de la revendication 2, où l'athérosclérose est le résultat d'une condition autoimmune.

5. Utilisation de la revendication 1 ou de la revendication 2, où l'athérosclérose résulte de la perte d'activité de GPI-PLD.

6. Utilisation d'une cellule hôte capable d'exprimer et de sécréter GPI-PLD dans la préparation d'un médicament pour le traitement de conditions **caractérisées par** l'athérosclérose.

7. Utilisation de l'une quelconque des revendications précédentes, où la condition est un trouble lipidique associé au diabète type 1, une condition **caractérisée par** une déficience en ApoA1, des troubles cardiovasculaires comme une maladie des artères coronariennes (CAD), le lupus érythématoseux généralisé (SLE) ou le syndrome de Sjogren.

8. Méthode de diagnostic d'un patient qui risque de développer une condition **caractérisée par** l'athérosclérose, la méthode consistant à déterminer la quantité de GPI-PLD et/ou l'activité de GPI-PLD dans un échantillon obtenu du patient.

9. Méthode de la revendication 8, où la méthode comprend les étapes de:
(a) mettre un échantillon obtenu du patient en contact avec un support solide où est immobilisé un agent de liaison ayant des sites de liaison spécifiques de GPI-PLD;
(b) déterminer la quantité de GPI-PLD ou l'activité de GPI-PLD qui se lie à l'agent de liaison.

10. Méthode de la revendication 9, où la méthode comprend de plus l'étape de:
(c) mettre en corrélation la valeur obtenue à l'étape (b) avec les mesures obtenues de sujets témoins pour déterminer si le patient a ou risque de développer la condition.

11. Méthode de l'une quelconque des revendications 8 à 10, où la condition est un trouble lipidique associé au diabète type 1, une condition **caractérisée par** une déficience en ApoA1, des troubles cardiovasculaires comme une maladie des artères coronariennes (CAD), le lupus érythématoseux généralisé (SLE) ou le syndrome de Sjogren.
